# EUROPEAN PATENT APPLICATION

(11) **EP 1 449 435 A1**
(43) Date of publication of application: **25.08.2004**
(21) Application number: 02773008.4
(22) Date of filing: 24.10.2002
(51) Int. Cl.: A01N 43/90, A01N 43/56, A01N 43/60, A61K 31/35, A61K 31/415, A61K 31/4184, A61K 31/4985, A61K 31/55, A61P 33/00, A61P 33/10, A61P 33/14

(54) **ANTHELMINTIC COMPOSITION**

(30) Priority: 25.10.2001 JP 2001327198
(71) Applicant: Sankyo Lifetech Company, Limited, Tokyo 113-0033 (JP)
(72) Inventor: SAITO, Akio, Sankyo Company, Limited, Shinagawa-ku, Tokyo 140-8710 (JP); SUGIYAMA, Yoko, Sankyo Company, Limited, Shinagawa-ku, Tokyo 140-8710 (JP); TOYAMA, Toshimitsu, Sankoo Company, Limited, Shinagawa-ku, Tokyo 140-8710 (JP); NANBA, Toshihiko, Sankyo Company, Limited, Shinagawa-ku, Tokyo 140-8710 (JP)
(74) Representative: Gibson, Christian John Robert
(86) International application number: PCT/JP2002/011067
(87) International publication number: WO 2003/034825

(57) **Abstract**

An anthelmintic composition containing as the active ingredients the first component consisting of one or more members selected from among compounds represented by the general formura (I): 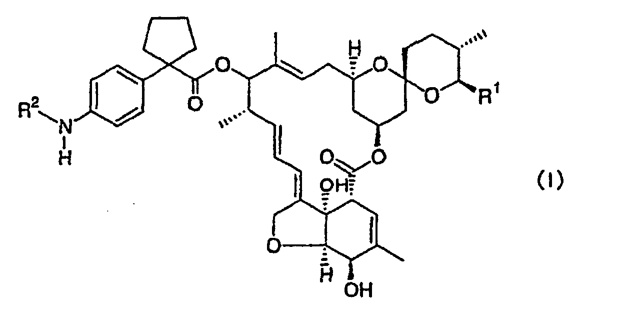 (wherein R¹ is methyl or the like; and R² is acetyl or the like) and so on and the second component consisting of one or more members selected from among praziquantels and so on.

## Description

### (Technical Field)

The present invention relates to an anthelmintic composition comprising milbemycin derivatives (the first ingredient) and the second ingredient as active ingredient; and a method for using it.

### (Background Art)

It has been known that Milbemycin or avermectin series compounds have excellent insecticidal activities against wide range of parasites, however, the milbemycin derivative represented by the following formula (I) of the present invention, a composition comprising thereof and bioactivities thereof have been conventionally unknown.

### (Disclosure of the Invention)

The inventors of the present invention have conducted extensive studies in order to obtain a composition exhibiting a strong activity against a wide range of endo- and ectoparasites including ectoparasites such as fleas, parasites in the digestive tract such as ascarides, and endoparasites such as filariae in both oral administration and local administration, and as a result, they have found that a composition comprising a specific milbemycin derivative and a second ingredient such as praziquantel, as active ingredients, exhibits an excellent anthelmintic activity, and the present invention has been accomplished.

The present invention relates to an anthelmintic composition comprising one or more kinds of first ingredient selected from milbemycin derivatives composed of a compound represented by formula (I): 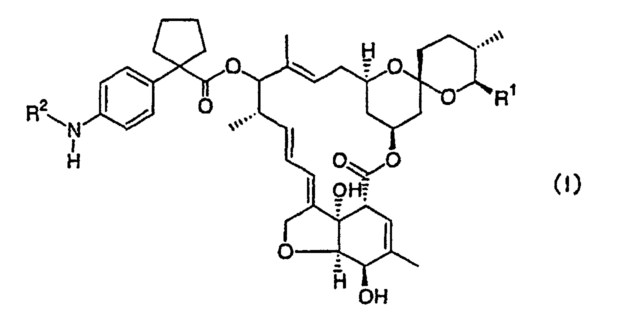 [wherein, R¹ represents a methyl group, an ethyl group, an isopropyl group or an s-butyl group; and R² represents an acetyl group, a methoxyacetyl group, a trifluoroacetyl group, a cyanoacetyl group or a methanesulfonyl group] or salts thereof, and, one or more kinds of second ingredients selected from the group consisting of praziquantels, fipronils, benzimidazoles and neonicotinoides, as active ingredients; and a method for using it.

The compound which is an active ingredient of the present invention and is represented by the above formula (I), is a compound exhibiting a strong activity against a wide range of endo- and ectoparasites including ectoparasites such as fleas, parasites in the digestive tract such as ascarides, and endoparasites such as filariae both in a oral administration and in a local administration.

In compound (I) which is an active ingredient of the present invention, (1) R¹ is preferably a methyl group or an ethyl group, and (2) R² is preferably a methoxyacetyl group.

In compound (I) which is an active ingredient of the present invention, it is preferable that R¹ is a methyl group or an ethyl group, and R² is a methoxyacetyl group.

As representative compounds of compound (I) which are active ingredients of the present invention, for example, the compounds described in the following Table can be cited, however, compound (I) is not limited to these compounds.

Incidentally, in the Table, "Me" represents a methyl group, "Et" represents an ethyl group, "iPr" represents an isopropyl group and "sBu" represents a s-butyl group, respectively.

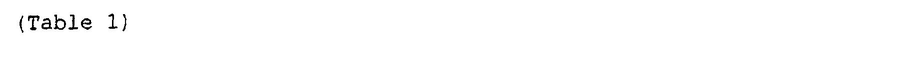

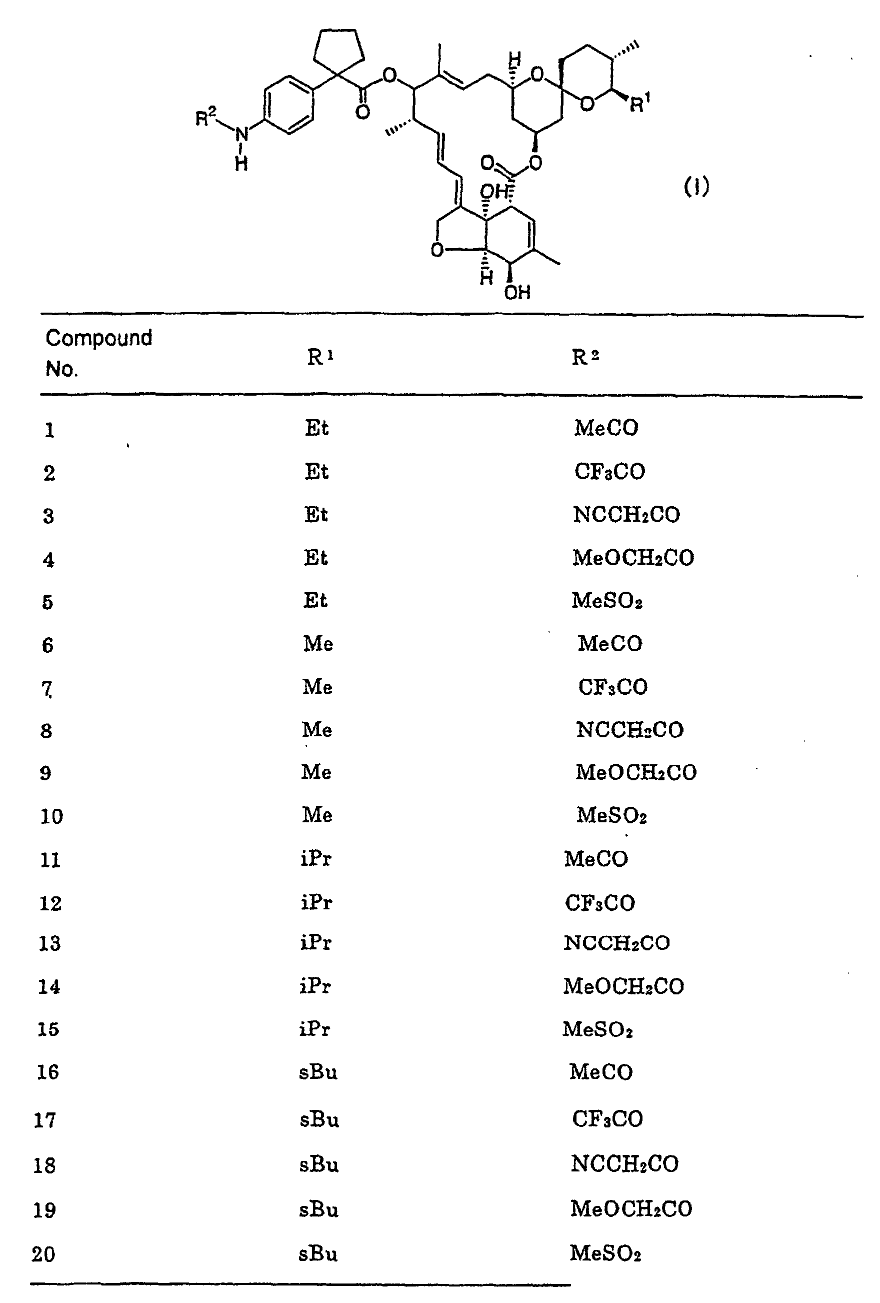

In compound (I) which is an active ingredient of the present invention, preferable compounds are compounds of exemplified compound number 1, 2, 3, 4, 5, 6, 7, 9, 10, 13, 14, 15, 16, 17, 19 and 20, more preferably compounds of exemplified compound number 1, 3, 4, 5, 6, 7, 9 and 10, and further more preferably compounds of exemplified compound number 1, 3, 4, 5, 9 and 10.

When compound (I) which is an active ingredient of the present invention or the salt thereof has an asymmetric carbon atom within the molecule, a stereoisomer which is the R configuration or the S configuration may be present, and both the individual isomer and compound thereof in any proportion is included in the present invention. Such a stereoisomer can be, for example, synthesized by using an optically-separated starting compound or optically separating synthesized compound (I) using a usual optical separation method or isolation method, if desired.

The compound (I) which is an active ingredient of the present invention or the salt thereof can be formed into a solvate, and such a solvate salt is also included in the active ingredient of the present invention. For example, compound (I) may absorb water when left in the atmosphere or when recrystallized to get absorbed water or to form a hydrate, and salt of such compound containing water is also included in the milbemycin derivatives which are active ingredients of the present invention.

The praziquantels which are active ingredients of the present invention are compound effective against Cestoidea on which milbemycins or avermectins have less effect (Parasitorogy Today, Vol 1, No 1, 10-17, 1985), and are compounds represented by the following formula: 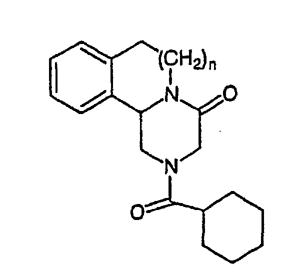 [wherein when n is 1, the compound is a praziquantel , and when n is 2, the compound is an epsiprantel].

The fipronils which are active ingredients of the present invention are compounds having an instantaneous effect against arthropod such as insects and mites, for example, fipronil (see, Japanese Provisional Patent Publication No. 63-316771) represented by the following formula: 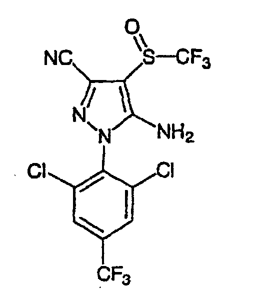 vaniliprole (see, Japanese Provisional Patent Publication No. 10-338678) represented by the following formula; and 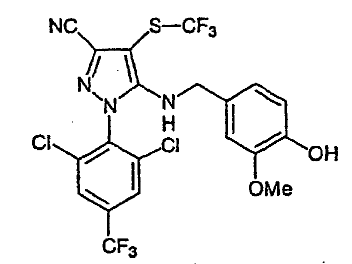 a compound represented by the following formula. 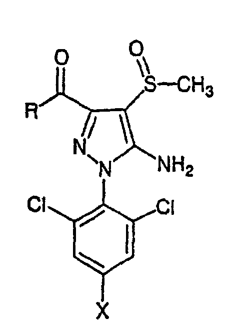 [wherein when R is a methyl group and X is a chlorine atom, the compound is acetprole, and when R is an ethyl group and X is a trifluoromethyl group, the compound is ethiprole] are known. Other than these, the compounds described in WO98/40359, WO98/02042, WO98/45274, WO98/28277, WO98/28278, WO98/28279, WO99/31070, WO00/62616, WO01/07413, or WO01/00614 can be raised. Among these, preferred are fipronil and vaniliprole.

Benzimidazoles which are active ingredients of the present invention, are used as anthelmintics against parasites in the digestive tract and, for example, albendazole, flubendazole and mebendazole are known (The benzimidazole anthelmintics-chemistry and biological activity, S. Sharma and S. Auzar, Progress in Drug Research Vol 27, 85-161).

As for neonicotinoides which are active ingredients of the present invention, for example, imidacloprid, thiamethoxam, nitenpyram and acetamiprid are known. Among these, imidacloprid is used as a flea exterminator for the local administration similarly to fipronils.

The second ingredient of the present invention is preferably praziquantels or fipronils, more preferably praziquantel, epsiprantel or fipronil.

Milbemycin derivatives which are active ingredients of the present invention can be prepared by the method of the steps A to D shown below. 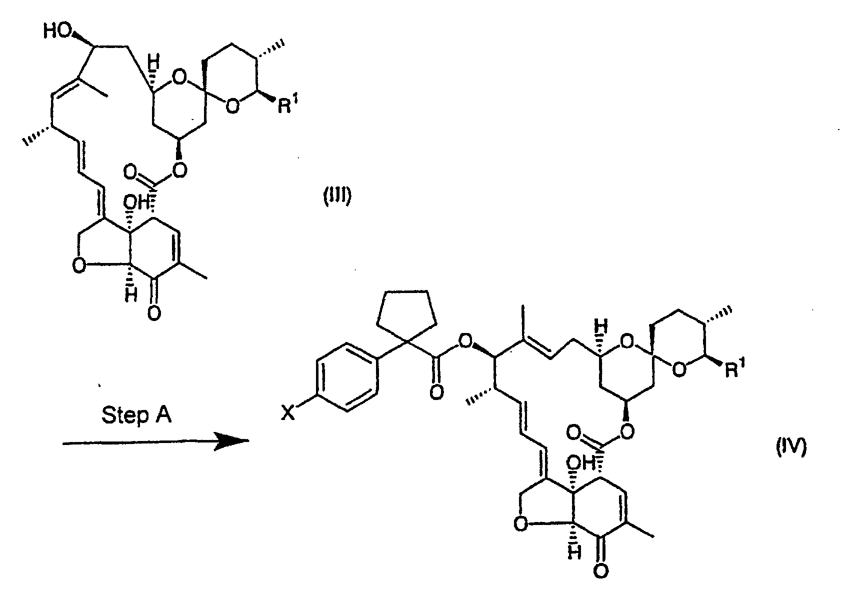 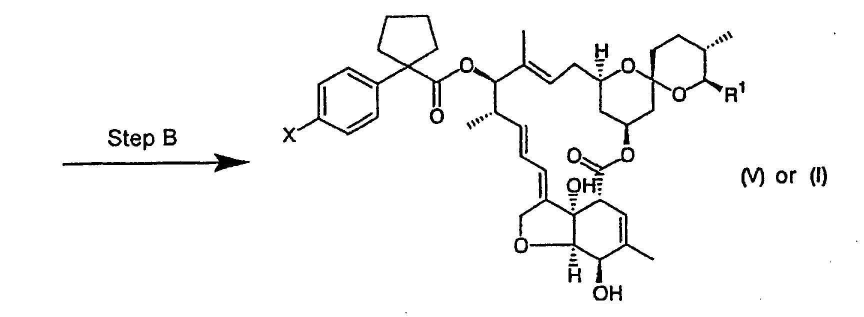 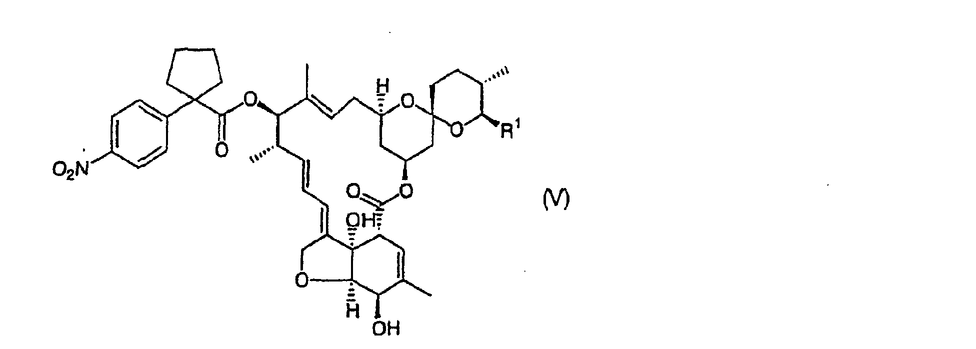 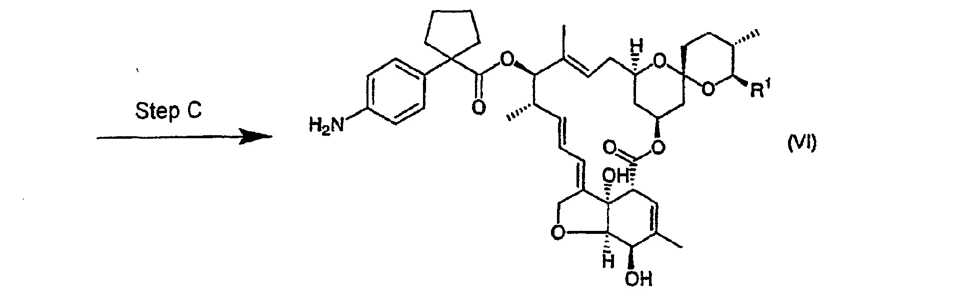 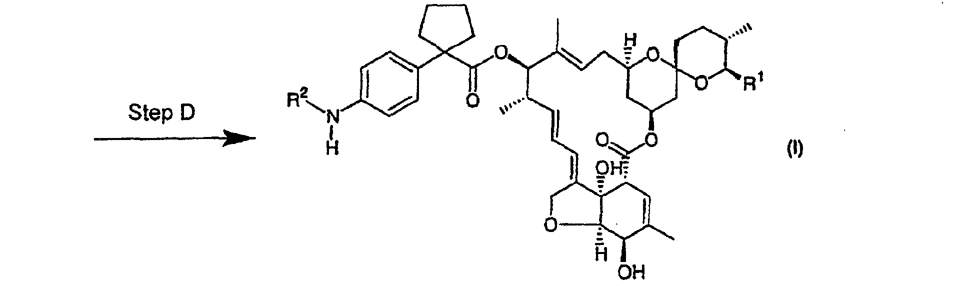 wherein R¹ and R² represent the same meaning as described above, and X represents a nitro group or a group represented by formula -NR²H.

15-Hydroxy milbemycin derivative having formula (III) as starting compound is a publicly known compound described in Japanese Provisional Patent Publication No. 60-158191.

### Step A

Step A is a step of reacting a compound (III) with a carboxylic acid represented by formula: 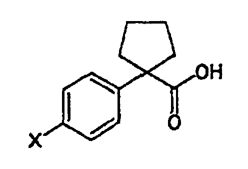 in an inactive solvent in the presence of trifluoromethanesulfonic acid which is a strong organic acid or trimethylsilyl trifluorosulfonate to produce a compound represented by formula (IV).

The amount of trifluoromethanesulfonic acid or trimethylsilyl trifluorosulfonate to be used is a catalytic amount in principle, and it does not need an equivalent weight with respect to compound (III). However, the amount may vary drastically depending upon the reactivity of the carboxylic acid employed.

Furthermore, when a powder of an inorganic compound is added to the reaction system, some reaction would be accelerated and a good result would be given. As such inorganic compounds, metal salts such as copper trifluoromethanesulfonate, cuprous iodide, zinc iodide, cobalt iodide and nickel iodide; Celite; silica gel and alumina can be cited, a copper salt such as copper trifluoromethanesulfonate or cuprous iodide is preferable, and cuprous iodide is the most preferable.

When the carboxylic acid to be used is hardly soluble in a solvent, a silyl ester of a carboxylic acid can be used. In this case, there can be used a method where the carboxylic acid is reacted with an equivalent amount of allyltrimethylsilane in the presence of trifluoromethanesulfonic acid or trimethylsilyl ester thereof as a catalyst and compound (III) is added to the obtained solution of trimethylsilyl ester.

The solvent to be used in the reaction is not particularly limited as long as it does not inhibit the reaction and it dissolves the starting materials to some extent. Suitably, aromatic hydrocarbons such as benzene, toluene and xylene; and halogenated hydrocarbons such as methylene chloride, 1,2-dichloroethane and chloroform can be raised.

The reaction temperature is from -10°C to 100°C, preferably from 0°C to 50°C. The reaction time varies mainly, depending on the reaction temperature, the starting compounds or the sort of the solvent to be used, and it is generally from 5 minutes to 6 hours, preferably from 10 minutes to 2 hours.

### (Step B)

Step B is a step of reacting compound (IV) with a reducing agent such as sodium borohydride in an inactive solvent to convert the carbonyl group at 5-position into a hydroxy group to produce a compound represented by formula (V) or compound (I) which is an active ingredient of the present invention.

The reducing agent to be used is not particularly limited as long as it is a reducing agent known as reducing the carbonyl group to a hydroxy group, for example, it can be a metal borohydride such as sodium borohydride or lithium borohydride, preferably sodium borohydride.

As a reactive solvent, any can be used without particular limitation as long as it is not involved in the reaction, and particularly the use of a lower alcohol such as methanol, ethanol or propanol; or ethers such as tetrahydrofuran or dimethoxyethane is preferable.

The reaction temperature is normally from 0°C to 50°C, and the reaction time is normally from 1 hour to 10 hours.

### (Step C)

Step (C) is a step of reducing the nitro group of compound (V) in an inactive solvent to produce a compound having an amino group, which is represented by formula (VI).

For the reduction of the nitro group, a method usually used can be used. As one of such methods, the catalytic reduction using a precious metal catalyst can be raised. As preferred examples for the catalyst employed in the reaction, palladium-on-carbon, palladium-on-barium sulfate, platinum oxide and so forth can be raised.

As a suitable solvent to be used in the reaction, an alcohol such as methanol or ethanol, an ether such as tetrahydrofuran or dioxane, and an ester such as ethyl acetate, can be raised.

The reaction temperature is normally from 10°C to 80°C, and the reaction time is normally from 10 minutes to 5 hours.

As another preferred reduction method, reduction with zinc powder in a solvent of acetic acid can be cited.

The reaction temperature is normally from 0°C to room temperature, and the reaction time is normally from 30 minutes to 12 hours.

As a more preferred reduction method, reduction with sodium borohydride in the presence of a nickel catalyst can be cited.

As a nickel catalyst, a nickel salt such as nickel chloride or nickel bromide can be used. Preferred are triphenylphosphine complexes of these nickel salts.

As suitable solvents to be used in the reaction, for example, alcohols such as methanol or ethanol, and ethers such as tetrahydrofuran or dioxane can be cited.

The reaction temperature is normally from 0°C to room temperature, and the reaction time is normally about from 10 minutes to 120 minutes.

### (Step D)

Step D is a step of reacting an amino group of the compound represented by compound (VI) with an acid represented by formula R²-OH (wherein R² represents the same meaning as mentioned above) or reactive derivatives thereof in an inactive solvent to produce a compound represented by compound (I) which is an active ingredient of the present invention.

As reactive derivatives of the acid represented by the formula R²-OH, what is usually used in a condensation reaction, such as acid halides (acid chloride, acid bromide, etc.), an acid anhydride, a mixed acid anhydride, an active ester or an active amide, can be cited.

When the acid represented by formula R²-OH is used, a dehydrating agent such as dicyclohexylcarbodiimide (DCC), 2-chloro-1-methylpyridinium iodide, p-toluenesulfonic acid or sulfuric acid is used, and preferably such a dehydrating agent is 2-chloro-1-methylpyridinium iodide. The amount of dehydrating agent employed is normally from 1 to 5 equivalents, preferably from 1 to 2 equivalents, to the acid represented by the formula R²-OH.

The solvent to be used is not particularly limited as long as it does not inhibit the reaction and it dissolves the starting material to some extent, for example, it can be a hydrocarbon such as hexane, petroleum ether, benzene or toluene; a halogenated hydrocarbons such as chloroform, methylene chloride or 1,2-dichloroethane; an ether such as ethyl ether or tetrahydrofuran; an amide, such as N,N-dimethylformamide; a sulfoxide such as dimethyl sulfoxide; a nitrite such as acetonitrile; and a mixture of these solvents. Preferred is dichloromethane or 1,2-dichloroethane.

The reaction temperature is usually from -70°C to 90°C, preferably from 0°C to 60°C. The reaction time varies mainly according to the reaction temperature, the starting compounds, reaction reagents or the sort of the solvent employed, and it is normally from 15 minutes to a whole day and night, and preferably from 30 minutes to 6 hours.

When the above-described reactive derivative is an acid halide, the reaction is preferably carried out in the presence of a base. As preferred bases, for example, organic bases such as triethylamine, N,N-dimethylaniline, pyridine, 4-dimethylaminopyridine, 1,5-diazabicyclo[4.3.0]nonene-5 (DBN) and 1,8-diazabicyclo[5.4.0]undecene-7 (DBU) can be raised.

The amount of the acid halide to be used is normally from 1 to 10 equivalents, and that of the base employed is normally from 2 to 8 equivalents, to compound (VI).

The solvents used in the reaction, the reaction temperature, the reaction time and the like each is the same as that when a carboxylic acid itself is used.

The reaction temperature is normally from 0°C to 50°C, and the reaction time is normally from 5 minutes to 2 hours.

After completion of each step described above, each desired compound, the compound (IV), (V) and (I) are isolated from the reaction mixture by a commonly known method, and, if necessary, purified by a known method, such as the column chromatography.

Natural milbemycins and derivatives thereof, which are starting materials of compound (III), are fermentation products and may be any of a single compound or a mixture thereof. Therefore, compound (I) can also be prepared as a single compound or as a mixture thereof.

A milbemycin derivative which is an active ingredient of the present invention can be mixed with a second ingredient which is another active ingredient in a certain ratio and can be prepared to a formulation suitable for oral administration or local administration.

As combinations of the milbemycin derivatives and the second ingredient, compound (I) and praziquantels, compound (I) and fipronils, and compound (I), praziquantels and fipronils, can be raised. Further, when a plurality of compounds are used as the second ingredient, the blending ratio of the compounds can be varied depending on the combination method and the intended purpose. For example, there can be used a combination that fipronil is 0.2 and praziquantel is 0.5 in terms of a weight ratio, when compound (I) is recognized as 1.

In the composition of the present invention, the blending ratio of the second ingredient to compound (I) varies depending on the sort and the use of the second ingredient to be used. For example, when the second ingredient is fipronil, the ratio is normally from 0.1 to 5, preferably from 0.2 to 1.0. When the second ingredient is praziquantel, the ratio is normally from 0.1 to 2, preferably from 0.2 to 0.5. When the second ingredient is benzimidazole, the ratio is normally from 1.0 to 100, preferably from 2.0 to 20. When the second ingredient are neonicotinoide, the ratio is normally from 0.2 to 10, preferably from 0.5 to 2.0. In addition, when a plurality of compounds are used as the second ingredient, the blending ratio of each ingredient can be prepared to be within the above-described range, respectively.

When the composition of the present invention is used as an endo- and ectoanthelmintics in an animal or a human, it can be administered orally as a liquid drink. The liquid drink is an appropriate non-toxic solvent or solution in water, suspension or dispersion with a suspending agent such as bentonite and a wetting agent or other excipients. The liquid drink, in general, may also contain an anti-foaming agent. The content of the active component in a drink formulation such as liquid drink is usually from 0.01 to 0.5% by mass, preferably from 0.01 to 0.1% by mass.

When it is desired to administer orally the composition of the present invention in a unit dosage form as a dried solid, capsules, pills or tablets, containing the desired amount of the active ingredient are normally employed. These dosage forms are prepared by mixing the active ingredient uniformly with suitable pulverized diluents, fillers, disintegrators and/or binding agents, for example starch, lactose, talc, magnesium stearate, vegetable gum and the like. Such unit dosage formulations may vary widely concerning the weight and contents of the insecticide depending upon the species of the host animal to be treated, the degree of the infection, the species of the parasite and the body weight of the host.

When a composition of the present invention is administered by animal feedstuffs, the composition is dispersed uniformly in the feedstuffs, used as a top dressing or used in the form of pellets. The content of active ingredient in the final feedstuff is usually from 0.0001 to 0.02% by mass, in order to achieve the desired anthelmintic effect.

Further, a product dissolved or dispersed in a liquid carrier excipient can be administered to an animal parenterally into the proventriculus, the muscle or the trachea or by a hypodermic injection. For parenteral administration, the active ingredient is preferably mixed with a suitable vegetable oil such as peanut oil or cottonseed oil. The content of the active ingredient in such formulation is generally from 0.05 to 50% by weight.

Further, another desirable administration form is a method where preparations are dissolved in a solvent and administered directly to a local site. As for such a solvent, the use of an alcohol such as ethanol, isopropanol, oleyl alcohol or benzyl alcohol; a carboxylic acid such as lauric acid or oleic acid; an ester such as ethyl lactate, isopropyl myristate or propylene carbonate; a sulfoxide such as dimethylsulfoxide; or an amide such as N-methylpyrrolidone, which are known to heighten percutaneous absorptivity, individually or as mixed solvent thereof, is preferable.

In the present invention, the amount of the active ingredient to be used is varied depending upon the species of the animal to be treated, and the type and degree of parasitic infection, and it is usually from 0.01 to 100 mg, preferably from 0.5 to 50.0 mg, per 1 kg of body weight of the animal, and oral administration is desirable. Such a dose can be administrated in a single dose or in divided doses over a relatively short period such as from 1 to 5 days.

The composition of the present invention has a high anthelmintic activity against wide range of endo- or ectoparasites and exhibits excellent control effects against various diseases caused by insects and the parasites being parasitic on animals.

That is, the composition of the present invention exerts an excellent insecticidal effect against fleas being parasitic on a pet or a human, and is extremely effective as anthelmintics against them.

As fleas to be targeted, Ctenocephalides felis, Ctenocephalides canis and so forth can be raised.

In the field of veterinary medicines, the composition of the present invention can be used against various harmful parasites of animals (endo- and ectoparasites), for example, insects and helminthes. As examples of these parasites of animals, Gasterophilus spp., Stomoxys spp., Trichodectes spp., Rhodnius spp., Hypoderma spp., Oestrus spp., Haematopinus spp and so forth can be raised. Further, the composition of the present invention has an excellent miticidal activity against Ixodidae, Dermanyssid-ae, Sarcoptidae, Argasidae, Dermanyssid and Psoroptidae, which are parasitic on animals.

The composition of the present invention has excellent parasiticidal activity as an anthelmintic for an animal and a human, and in particular, is effective against nematodes which infect livestock, poultry and pet animals such as pigs, sheep, goats, cows, horses, dogs, cats and fowl. As such nematodes, for example, Haemonchus, Trichostrongylus, Ostertagia, Nematodirus, Cooperia, Ascaris, Bunostomum, Oesophagostomum, Chabertia, Trichuris, Storongylus, Trichonema, Dictyocaulus, Capillaria, Heterakis, Toxocara, Ascaridia, Oxyuris, Ancylostoma, Uncinaria, Toxascaris, and Parascaris can be raised. In particular, certain species of the genera Nematodirus, Cooperia and Oesophagostomum attack the intestines, while those of the genera Haemonchus and Ostertagia parasitize the stomach, and parasites of genus Dictyocaulus can be found in the lungs, and the composition of the present invention also exhibits the activity against these. Further, parasites belonging to the families Filariidae and Setariidae can be found in other tissues or organs such as the heart, blood vessels and subcutaneous and lymphatic tissues. The composition of the invention also exhibits the activity against these parasites.

The composition of the present invention exhibits activity also against cestoid and flukes. As the cestoids, for example, Dipylidium caninum, Taenia taeniaeformis, Taenia solium, Taenia saginata, Hymenolepis diminuta, Moniezia benedeni, Diphyllobothrium latum, Diphyllobothrium erinacei, Echinococcus granulosus, and Echinococcus multilocularis can be raised and as the flukes, Fasciola hepatica, F.gigantica, Paragonimus westermanii, Fasciolopsic bruski, Eurytrema pancreaticum, E.coelomaticum, Clonorchis sinensis, Schistosoma japonicum, S.haematobium, and S.mansoni can be raised.

The composition of the present invention is also useful against parasites which infect a human, and as parasites of human digestive, for example, Ancylostoma, Necator, Asdaris, Strongyloides, Trichinella, Capillaria, Trichuris, and Enterobius can be raised.

The compound of the present invention also exhibits the activity against parasites of the genera Wuchereria, Brugia, Onchoceca, Dirofilaria, Loa, and Dracunculidae of the family Filariidae which are found in blood or other tissues and organs other than the digestive tract and are medically important, parasites of the genus Dracunclidae of the family Deacunculus and parasites of the genera Strongyloides and Trichinella, which in a particular state may parasitize outside the intestinal tract, although they are essentially intestinal parasites.

### (Best Mode for Carrying out the Invention)

Although the composition of the present invention is explained below in more detail by referring to Reference Examples and Test Examples, the composition of the present invention is not limited thereto.

### [Reference Example 1]

13-[1-(4-methoxyacetylaminophenyl) cyclopentanecarbonyloxy]-5-hydroxy-milbemycin A4 (I: R¹=Et, R²=MeOCH₂CO, Compound No. 4)

### (1) 13-[1-(4-Nitrophenyl)cyclopentanecarbonyloxy]-5-oxomilbemycin A4 (IV: R¹=Et, X=NO₂, Step A)

Trifluoromethanesulfonic acid (0.18 ml) were added to a suspension of 1-(4-nitrophenyl)cyclopentanecarboxylic acid (10.13 g, 40.0 mmol) in dichloromethane (150 ml) and stirred for 20 minutes in a nitrogen stream. To the obtained transparent reaction solution, 15-hydroxy-5-oxomilbemycin A4 (5.57 g, 10.0 mmol) were added and further continuously stirred for 30 minutes. After the completion of reaction, the reaction solution was poured into 4% aqueous sodium hydrogencarbonate solution and then extracted with ethyl acetate. The extracted solution was washed with a 4% aqueous sodium hydrogencarbonate solution and water, dried over magnesium sulfate and then, the solvent was removed. The obtained residue containing 13-[1-(4-nitrophenyl)cyclopentanecarbonyloxy]-5-oxomilbemycin A4 was used in the next step without further purification.

### (2) 13-[1-(4-Nitrophenyl)cyclopentanecarbonyloxy]-5-hydroxymilbemycin A4 (V: R¹=Et, Step B)

The 5-oxo derivative obtained in the Step A was dissolved in methanol (200 ml), to which sodium borohydride (1.52 g, 40 mmol) and boron trifluoride·ether adduct (two drops) were added at -40°C and stirred for 10 minutes. After the completion of reaction, ethyl acetate (400 ml) was added. The mixed solution was washed three times with water and then dried over anhydrous sodium sulfate. The solvent was removed under a reduced pressure. The obtained residue was purified by chromatography on a silica gel column (eluant: ethyl acetate/hexane=1/1) to give 6.79 g of 13-[1-(4-nitrophenyl)cyclopentanecarbonyloxy]-5-hydroxymilbemycin A4.

NMR Spectrum (400MHz) δ (ppm): 8.16(2H, d,7.9Hz), 7.53(2H, d, J=7.9Hz), 5.70-5.90(2H, m), 5.37(1H, s), 5.25-5.40(3H, m), 4.84(1H, d, J=10.6Hz ), 4.67 and 4.63(2H, AB-q, J=14.4Hz), 4.28(1H, m), 4.07(1H, s), 3.94(1H, d, J=6.4Hz), 3.55(1H, m), 3.25(1H, m), 3.02(1H, m), 1.88(3H, s), 1.29(3H, s), 0.99(3H, t, J=7.3Hz), 0.82(3H, d, J=6.5Hz), 0.77(3H, d, J=6.4Hz).

### (3) 13-[1-(4-Aminophenyl)cyclopentanecarbonyloxy]-5-hydroxymilbemycin A4 (VI: R¹=Et, Step C)

The nitro derivative obtained in (2) was dissolved in ethyl acetate (100 ml), and Methanol (100 ml) and a triphenylphosphine complex of nickel (II) chloride (1.12 g, 1.7 mmol) were added to the solution, to which sodium borohydride (100 mg, 2.6 mmol) were gradually added over 10 minutes under ice cooling. After being stirred for 10 minutes, ethyl acetate (70 ml) was added to the reaction solution, and which was then washed three times with water and dried over anhydrous sodium sulfate, and the solvent was then removed under reduced pressure. The obtained residue was crystallized by adding ethyl acetate hexane to give 5.87 g of 13-[1-(4-aminophenyl)cyclopentanecarbonyloxy]-5-hydroxymilbemycin A4 as a crystal.

Melting Point: 235-239°C

NMR Spectrum (400MHz) δ (ppm): 7.16(2H, d, J=8.6Hz), 6.65(2H, d, 8.6Hz), 5.77-5.84(2H, m), 5.44(1H, s), 5.32-5.43(3H, m), 4.84(1H, d, J=10.5Hz ), 4.70 and 4.73(2H, AB-q, J=14.5Hz), 4.33(1H, m), 4.13(1H, s), 4.00(1H, d, J=6.2Hz), 3.54(1H, m), 3.30(1H, m), 3.09(1H, m), 1.92(3H, s), 1.36(3H, s), 1.01(3H, t, J=7.3Hz), 0.87(3H, d, J=6.5Hz), 0.82(3H, d, J=6.5Hz).

### (4) 13-[1-(4-Methoxyacetylaminophenyl)cyclopentanecarbonyloxy]-5-hydroxymilbemycin A4 (I: R¹=Et, R²=MeOCH₂CO, Compound No. 4, Step D)

The amino derivative obtained in (3) was dissolved in tetrahydrofuran (60 ml), followed by adding pyridine (0.52 ml, 6.4 mmol) and methoxyacetyl chloride (0.58 ml, 6.2 mmol) at -30°C, and stirring for 10 minutes. After the completion of reaction, ethyl acetate (250 ml) was added to the reaction solution, which was washed successively with 0.5 mol/L citric acid solution, water, 4% aqueous sodium hydrogencarbonate solution and water, and then dried over anhydrous sodium sulfate, and the solvent was then removed under a reduced pressure. The obtained residue was purified by chromatography on a silica gel column (eluant: ethyl acetate/hexane=2/1) to give 4.38 g of the desired compound.

NMR Spectrum (400MHz) δ (ppm): 8.20(1H, s), 7.49(2H, d, J= 8.6Hz), 7.29(2H, d, J=8.6Hz), 5.77(1H, m), 5.74(1H, m), 5.39(1H, s), 5.27-5.37(3H, m), 4.80(1H, d, J=10.5Hz ), 4.68 and 4.64(2H, AB-q, J=14.5Hz), 4.28(1H, m), 4.08(1H, s), 4.01(2H, s), 3.95(1H, d, J=6.4Hz), 3.54(1H, m),3.51(3H, s), 3.25(1H, m), 3.01(1H, m), 2.64(2H, m), 2.32(1H, d, J=8.1Hz), 1.87(3H, s), 0.96(3H, t, J=7.3Hz), 0.82(3H, d, J=6.5Hz), 0.76(3H, d, J=6.5Hz).

### [Reference Example 2]

### 13-[1-(4-Acetylaminophenyl)cyclopentanecarbonyloxy]-5-hydroxymilbemycin A4 (I: R¹=Et, R²=MeCO, Compound No. 1)

In the same manner as in Reference Example 1 (4) except for using acetic anhydride in place of methoxyacetyl chloride in Reference Example 1 (4), the title compound was produced (yield in the Step D: 89.8%).

NMR Spectrum (400MHz) δ (ppm): 7.41(2H, d, J=8.6Hz), 7.27(2H, d, 8.6Hz), 5.70-5.80(2H, m), 5.39(1H, s), 5.25-5.40(3H, m), 4.80(1H, d, J=10.6Hz ), 4.64 and 4.68(2H, AB-q, J=14.2Hz), 4.28(1H, dd, J=6.3 and 8.4Hz), 4.07(1H, s), 3.95(1H, d, J=6.3Hz), 3.54(1H, m),3.25(1H, m), 3.01(1H, m), 2.61(2H, 2), 2.33(1H, d, J=8.4Hz), 1.87(3H, s), 1.58(3H, s), 0.96(3H, t, J=7.3Hz), 0.82(3H, d, J=6.5Hz), 0.76(3H, d, J=6.5Hz).

### [Reference Example 3]

### 13-[1-(4-Cyanoacetylaminophenyl)cyclopentanecarbonyloxy]-5-hydroxymilbemycin A4 (I: R¹=Et, R²=NCCH₂CO, Compound No. 3)

In the same manner as in Reference Example 1 (4) except for using cyanoacetyl chloride in place of methoxyacetyl chloride in Reference Example 1 (4), the title compound was produced (yield in the Step D: 89.8%).

NMR Spectrum (400MHz) δ (ppm): 7.68(1H, s), 7.42(2H, d, J=8.7Hz), 7.33(2H, d, 8.7Hz), 5.72-5.77(2H, m), 5.39(1H, s), 5.28-5.37(3H, m), 4.81(1H, d, J=10.6Hz), 4.64 and 4.68(2H, AB-q, J=14.5Hz), 4.29(1H, m), 4.07(1H, s), 3.95(1H, d, J=6.2Hz), 3.55(2H, s), 3.52(1H, m), 3.25(1H, m), 3.01(1H, m), 2.62(2H, 2), 2.32(1H, d, J=8.2Hz), 1.87(3H, s), 0.96(3H, t, J=7.3Hz), 0.82(3H, d, J=6.5Hz), 0.76(3H, d, J=6.5Hz).

### [Reference Example 4]

### 13-[1-(4-Methanesulfonylaminophenyl)cyclopentanecarbonyloxy]-5-hydroxymilbemycin A4 (I: R¹=Et, R²=MeSO₂, Compound No. 5)

In the same manner as in Reference Example 1 (4) except for using methanesulfonyl chloride in place of methoxyacetyl chloride in Reference Example 1 (4), the title compound was produced (yield in the Step D: 88.2%).

NMR Spectrum (400MHz) δ (ppm): 7.32(2H, d, J=8.7Hz), 7.14(2H, d, 8.7Hz), 6.47(1H, s), 5.70-5.80(2H, m), 5.39(1H, s), 5.25-5.37(3H, m), 4.80(1H, d, J=10.6Hz ), 4.64 and 4.68(2H, AB-q, J=14.0Hz), 4.28(1H, dd, J= 6.4 and 8.2Hz), 4.07(1H, s), 3.95(1H, d, J=6.4Hz), 3.54(1H, m),3.25(1H, m), 3.01(1H, m), 2.93(3H, s), 2.62(2H, 2), 2.33(1H, d, J=8.2Hz), 1.87(3H, s), 0.96(3H, t, J=7.3Hz), 0.82(3H, d, J=6.5Hz), 0.74(3H, d, J=6.5Hz).

### [Reference Example 5]

### 13-[1-(4-Methoxyacetylaminophenyl)cyclopentanecarbonyloxy]-5-hydroxymilbemycin A3 (I: R¹=Me, R²=MeOCH₂CO, Compound No. 9)

### (1) 13-[1-(4-Aminophenyl)cyclopentanecarbonyloxy]-5-hydroxymilbemycin A3

### (VI: R¹=Me, Steps A to C)

In the same manner as in Reference Examples 1 (1) to (3) except for using 15-hydroxy-5-oxomilbemycin A3 in place of 15-hydroxy-5-oxomilbemycin A4 in Reference Example 1, 13-[1-(4-aminophenyl)cyclopentanecarbonyloxy]-5-hydroxymilbemycin A3 was obtained.

NMR Spectrum (400MHz) δ (ppm): 7.11(2H, d, J=8.5Hz), 6.60(2H, d, 8.5Hz), 5.71-5.78(2H, m), 5.38(1H, s), 5.25-5.40(3H, m), 4.80(1H, d, J=10.6Hz ), 4.65 and 4.68(2H, AB-q, J=13.9Hz), 4.28(1H, m), 4.08(1H, s), 3.95(1H, d, J=6.1Hz), 3.59(2H, broad-s), 3.52(1H, m),3.18-3.26(2H, m), 2.57(2H, m), 1.87(3H, s), 1.31(3H, s), 1.13(3H, d, J=6.4Hz), 0.83(3H, d, J=6.5Hz), 0.77(3H, d, J=6.6Hz).
13-[1-(4-Methoxyacetylaminophenyl)cyclopentanecarbonyloxy]-5-hydroxymilbemycin A3 (I: R¹=Me, R²=MeOCH₂CO, Compound No. 9, Step D)

Using the amino derivative obtained in (1), the title compound was obtained in the same manner as in Reference Example 1 (4)(yield: 92.3%).

NMR Spectrum (400MHz) δ(ppm): 8.21(1H, s), 7.49(2H, d, J=8.6Hz), 7.29(2H, d, 8.6Hz), 5.71-5.78(2H, m), 5.38(1H, s), 5.24-5.36(3H, m), 4.80(1H, d, J=10.6Hz ), 4.64 and 4.68(2H, AB-q, J=13.7Hz), 4.28(1H, m), 4.07(1H, s), 4.01(2H, s), 3.95(1H, d, J=6.3Hz), 3.51(3H, s), 3.50(1H, m),3.18-3.26(2H, m), 2.61(2H, m), 1.87(3H, s), 1.28(3H, s), 1.13(3H, d, J=6.3Hz), 0.83(3H, d, J=6.6Hz), 0.76(3H, d, J=6.5Hz).

### [Test Example 1]

### Oral Test

Compound (5 mg/kg) of Reference Example 1 and praziquantel (1.25 mg/kg) were encapsulated into gelatine to a predetermined amount to prepare a formulation for Oral Test. Incidentally, formulations were prepared as controls by using each of the compound of Reference Example 1 solo and fipronil solo to carry out the test.

The formulation was administered orally once to dogs (two dogs in each test location) naturally infected with Dipylidium caninum and Toxocara canis. Incidentally, the infection with Dipylidium caninum was confirmed by the excretion of proglottid of Dipylidium caninum into faeces three days before administration of drugs. The infection with Toxocara canis was confirmed by the excretion of eggs of Toxocara canis into faeces immediately before administration of drugs. On the 10th day after the administration of drugs, the dogs were subjected to postmortem to count the number of surviving Dipylidium caninum and Toxocara canis.

As a result, the simultaneous use of the compound in Reference Example 1 and praziquantels was effective in exterminating two kinds of parasites of Dipylidium caninum and Toxocara canis. In contrast, the sole use of the compound in Reference Example 1 was effective in exterminating Toxocara canis, however, was ineffective in exterminating Dipylidium caninum. Further, the sole use of praziquantels was effective in exterminating Dipylidium caninum, however, was ineffective in exterminating Toxocara canis.

### [Test Example 2]

### Spot-on Test (extermination test on mites)

Compound (10g) of Reference Example 1 and fipronil (3.8g) were mixed. This mixture was dissolved in a mixed solvent (9:1) of benzyl alcohol and propylene carbonate so as to be a total volume of 100 ml, and 0.05% of BHT was further added to prepare a formulation for the spot-on test. Further, as a control, formulations were prepared by using each of the compound of Reference Example 1 solo and fipronil solo to carry out the test.

The above-described formulation for spot-on test was dropped to the back part of the neck of each dog (two dogs in each test location) naturally infected with mites at a dose of 5 mg/kg of the compound in Reference Example 1. In each dog, infection with mites was examined three days after the dropwise administration.

As a result, the simultaneous use of the compound in Reference Example 1 and fipronil was effective in treating infection with mites in spite of a small therapeutic dose in fipronil. In contrast, the sole use of the compound in Reference Example 1 was ineffective in treating infection with mites. Further, the sole use of fipronil where the therapeutic dose was small, was ineffective in treating infection with mites.

### [Test Example 3]

### Spot-on test (extermination test on fleas)

There was used beagles (4 to 7 months old, four dogs) previously infected with cat fleas to count the number of fleas collected from the surface of the body of each dog three days after infection and to determine the infection ratio (usually 70 to 90%) of each dog. In the same manner as in Test Example 2, the formulation for the spot-on test was administered dropwise to the interscapular of each dog at a dose of 5 mg/kg of the compound of Reference Example 1. On 7th day, 21th day and 28th day after the administration, each dog was infected with 100 cat fleas and the number of fleas collected from the surface of the body of the dog was counted on the third day from the infection. An extermination ratio was determined from a ratio between the collect ratios before and after administration.

As a result, no fleas were collected from the surfaces of the body of the dog in the tests on 7th day, 21th day and 28th day after the administration, and the extermination ratio was 100% even 28 days after the administration. In contrast, no significant shift of the infection ratio was observed for the four dogs in a group of no administration carried out simultaneously.

### (INDUSTRIAL APPLICABILITY)

The composition of the present invention has a high exterminating activity against wide range of endo- and ectoparasites and exhibits excellent control effects against various disease injuries caused by insects and the parasites being parasitic on animals.

## Claims

1. An anthelmintic composition comprising one or more kinds of first ingredients selected from milbemycin derivatives consisting of a compound represented by formula (I) : 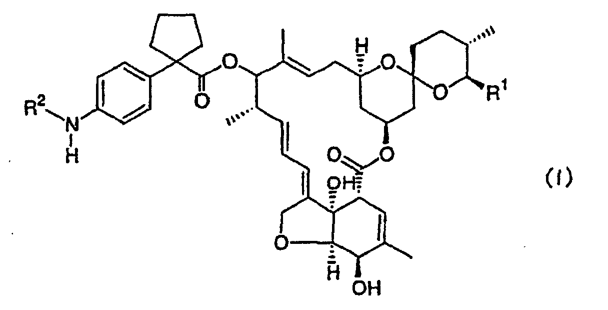 [wherein, R¹ represents a methyl group, an ethyl group, an isopropyl group or an s-butyl group; and R² represents an acetyl group, a methoxyacetyl group, a trifluoroacetyl group, a cyanoacetyl group or a methanesulfonyl group] or salts thereof, and one or more kinds of second ingredients selected from the group consisting of praziquantels, fipronils, benzimidazoles and neonicotinoides, as active ingredients.

2. The anthelmintic composition of claim 1, wherein R¹ is a methyl group or an ethyl group.

3. The anthelmintic composition of claim 1 or 2, wherein R² is a methoxyacetyl group.

4. The anthelmintic composition of any one of claims 1 to 3, wherein the second ingredients are praziquantels or fipronils.

5. The anthelmintic composition of any one of claims 1 to 3, wherein the second ingredients are praziquantel, epsiprantel or fipronil.

6. A method for exterminating endo- and ectoparasites by administering orally or locally the anthelmintic composition of any one of claims 1 to 5.
